# EUROPEAN PATENT SPECIFICATION

(11) **EP 1 337 549 B1**
(45) Date of publication and mention of the grant of the patent: **05.04.2006**
(21) Application number: 01990489.5
(22) Date of filing: 27.11.2001
(51) Int. Cl.: C07K 1/113, C07K 1/04

(54) **PROCESS FOR FOLDING CHEMICALLY SYNTHESIZED POLYPEPTIDES**
VERFAHREN ZUR RÜCKFALTUNG VON CHEMISCH HERGESTELLTEN POLYPEPTIDEN
PROCEDE DE PLIAGE DE POLYPEPTIDES CHIMIQUEMENT SYNTHETISES

(30) Priority: 27.11.2000 EP 00204207
(43) Date of publication of application: 27.08.2003
(60) Divisional application: 06075237.5
(73) Proprietor: RMF DICTAGENE S.A., 1066 Epalinges (CH)
(72) Inventor: VERDINI, Antonio, 47011 Castrocaro Terme (IT); CORRADIN, Giampietro, 1000 Lausanne 26 (CH); ROGGERO, Mario, 1066 Epalinges (CH)
(74) Representative: Van Someren, Petronella F. H. M.
(86) International application number: PCT/EP2001/014163
(87) International publication number: WO 2002/050099

(56) References cited:
- EP-A- 0 628 573
- WO-A-91/08762
- WO-A-95/06064
- DATABASE CA [Online] CHEMICAL ABSTRACTS SERVICE, COLUMBUS, OHIO, US; ROUX, PASCALE ET AL: "Comparison of the kinetics of S-S bond, secondary structure, and active site formation during refolding of reduced denatured hen egg white lysozyme" retrieved from STN Database accession no. 132:148471 CA XP002202938 & PROTEIN SCIENCE (1999), 8(12), 2751-2760 , 1999,
- PLANAS, ANTONI ET AL: "Sequential protection-modification method for selective sulfhydryl group derivatization in proteins having more than one cysteine" PROTEIN ENG. (1990), 3(7), 625-8 , 1990, XP002202937

## Description

The present invention relates to a process for folding chemically synthesized polypeptides. In addition, the invention relates to a process for producing biologically active proteins.

Over the last decade there has been an escalation in the demand of synthetic proteins after the successful chemical synthesis of fully active HIV-protease, a 99-residue enzyme prepared by highly optimized methods of solid-phase peptide synthesis (SPPS) based on the standard Boc/Bzl approach.

The synthesis in 1994 of crystalline ubiquitin, a small protein consisting of 76-residues, has further demonstrated that highly pure proteins can be synthesized by SPPS based on the Fmoc/t-Bu protocol, a method operationally simpler and chemically less complex than the Boc/Bzl procedure.

As of 2000, there is ample experimental evidence that single domain proteins containing between 60 and 100 amino-acid residues can be produced rapidly, reliably and economically by chemical synthesis, with the assistance of a peptide synthesizer, in amounts that are sufficient for structural and functional studies.

Proteins containing disulfide bridges prepared by chemical synthesis, once folded, have the same properties as natural and genetically engineered forms. Disulfide bridges of proteins form single or multiple intra- and/or interchain cyclic structures which impart considerable conformational restraints to the molecules, thus decisively contributing to the stabilization of the bioactive conformation.

Folded single domain proteins of known structure can be prepared by regioselective pairing of the cysteine residues. Various combinations of cysteine protecting groups compatible with the commonly used protection schemes were developed that allow for stepwise and pairwise deprotection and/or cooxidation of cysteine residues with complete selectivity.

However, a demonstration of how demanding is the chemistry involved for regioselective pairing of the cysteine residues in proteins containing multiple cystine residues is the recent synthesis of an insulin-like peptide, human relaxin. The synthesis of the A chain precursor was carried out using SPPS methods, the Fmoc/t-Bu approach and a p-alkoxybenzyl alcohol-based resin, while the B chain precursor was prepared using PAM resin (4-carboxyamidomethylbenzyl ester linkage to polystyrene-based resin) following the Boc/Bzl approach. Of the four cysteine residues of the A chain precursor, two were protected as the S-Trt (S-triphenylmethyl) derivatives, while the others had S-Acm (S-acetamidomethyl) and S-Meb (S-p-methylbenzyl) protection, respectively. The two cysteines of the B chain precursor were protected by S-Acm and S-Meb protecting groups. The intramolecular S-S bridge of the A chain was obtained first by iodine oxidation in AcOH. Then, the two intermolecular disulfide bridges connecting the A and B chains were obtained in two steps: in the first step, the free thiol of chain A precursor, obtained by HF deblocking of the S-Meb protecting group, was reacted with the activated Cys(Npys) (S-3-nitro-2-pyridine sulphenyl) residue of the B chain (directed intermolecular heterodisulfide formation) and, in the second step, the remaining S-S bridge was obtained by cooxidative removal of the S-Acm groups with iodine.

SPPS protocols offer the possibility to prepare by chemical synthesis a variety of polypeptides, containing the cysteine residues protected with the same blocking (protecting) group. Once the protecting groups are removed by a number of oxidizing reagents the disulfide bonds form directly. Polypeptides and/or proteins containing S-Trt or S-Acm protected cysteines can be indeed efficiently folded upon treatment with iodine, N-iodosuccinimide and cyanogen iodide under carefully controlled conditions of solvent, pH and reaction time that will minimize modification of oxidation sensitive Tyr, Met, and Trp and avoid overoxidation of cysteine thiols to the corresponding sulfonic acids.

Thallium(III) trifluoroacetate can replace the above oxidizing agents giving sometimes better yields of disulfide bonds. The major limitations of this reagent are its toxicity, the difficulty to remove thallium from the target polypeptide and the need to protect Met and Trp residues from oxidation.

Oxidizing reagents containing a mixture of sulfoxide/silyl compounds and trifluoroacetic acid have been successfully applied for the direct oxidation to disulfide bonds of polypeptide precursors containing S-Acm, S-But, S-Meb and S-Mob (S-p-methoxybenzyl) cysteine residues. The need to protect the Trp indole ring with formyl to avoid chlorination under oxidizing conditions is, however, the major limitation of this mixture.

Methods for the oxidative folding of linear, synthetic polythiol precursors (reduced polypeptide forms) are more popular and most frequently applied. In the simplest method, the appropriate disulfide bonds can be spontaneously formed in the presence of air or some other mild oxidizing agents. Furthermore, folding and cysteine pairing is obtained in the presence of both the reduced (RSH) and oxidized (R-S-S-R) forms of a low molecular weigth sulphydryl compound.

In synthetic polypeptides and small proteins consisting of a single domain the thermodynamic driving force for folding which results from a combination of H-bonding, ion pairing and hydrophobic effects is apparently substantial enough to spontaneously produce the native isomers in random renaturating oxidation.

From studies of the oxidative folding of multiple cysteine-containing small proteins, like enzyme inhibitors, toxins or hormones, useful informations have been derived about particular structural motifs e.g. cysteine-stabilized â-turn, cysteine-stabilized poly(Pro)-II helix fold and cysteine-stabilized á-â-structural fold, whose stabilization is the main driving force for the correct disulfide bridging even in relatively small peptide molecules. If attention is paid to the choice of buffers, temperature and additives that will stabilize the secondary structural motifs, then even complete correct folding of partially folded or scrambled (missfolded) proteins can be obtained in vitro.

A number of folding protocols for polythiol polypeptide species have been designed to minimize incorrect intramolecular cysteine pairing that leads to non-native, misfolded isomers and to avoid as much as possible random intermolecular disulfide bond formation that promote aggregation and precipitation.

Thus, air oxidation is generally carried out at high dilution of the precursor polythiol form (1mg/ml or below) under neutral or sligthly alkaline conditions. It usually requires a long duration and produces an harmless by-product as water in the reaction. Air oxidations are, however, difficult to control because trace amounts of metal ions strongly influence their rates. More important, basic and hydrophobic precursor molecules tend to aggregate and precipitate out of the solution at or near their basic or neutral isoelectric points during the folding process. Furthermore, side-products due to oxidation of Met accumulate during folding. Although the number of chemical operations necessary to fold polythiol precursors is reduced to a minimum, the disulfide bridges formation promoted by the molecular oxygen of air gives in many instances low yields, sometimes not occurring at all.

DMSO and potassium ferricyanide have also been used as oxidants. Potassium ferricyanide must, however, be used in the dark and, if Met and Trp are present in the polypeptide chain, oxidation side-products accumulate during folding. The use of DMSO often gives better results due to the fact that oxidative foldings can be conducted under acidic conditions at an efficient rate with no harmful products in the reaction. The method is particularly suitable for folding basic and hydrophobic polypeptide precursors due to the higher solubility characteristics of species undergoing oxidation in acidic buffers. However, problems in removing DMSO from the final product and reduction on the selectivity of disulfide bridges formation have been frequently reported. Furthermore, scrambling of disulfide bridges leading to misfolded isomers and oligomerization cannot be always avoided even with a careful control of the experimental conditions.

Higher yields of correct cysteine pairing and folding in small protein polythiol precursors are most frequently obtained by the use of redox buffers such as oxidized (GSSG) and reduced (GSH) glutathione and cystine/cysteine (Cys/Cys).

Thus, during the oxidative foldings of Ribonuclease A (R.R. Hantgan et al. Biochemistry 13, 613, 1974), the 49 amino acid core domain of Hirudin (B. Chatrenet and J.Y. Chang J. Biol. Chem. 267, 3038, 1992) and Bovine Pancreatic Trypsin Inhibitor (BPTI) (T. E. Creighton Methods Enzymol. 131, 83, 1986) induced by GSSG/GSH or Cys/Cys, free sulphydryls and disulfide groups are formed and reformed constantly throughout the folding process. Overall rates and yields are usually better than oxidative folding in air because thiol/disulfide exchange occurring through thiolate intermediates facilitates the reshuffling of non-native disulfide to the natural ones. As for oxidative folding in air, high dilution of the polythiol precursor is ' necessary to avoid aggregation, formation of oligomers and polymers and to maximize yields of the target protein species.

During the first stage of folding Hirudin¹⁻⁴⁹ in vitro, folding proceeds sequentially and irreversibly from the unfolded, reduced form (polythiol) to equilibrated isomers containing one and two disulfide bridges and to equilibrated species containing three disulfide bonds (scrambled isomers) (J. Y. Chang Biochem. J. 300, 643, 1994). Nearly all 75 possible protein species, including the native one, have been identified : 15 isomers with one S-S bridge, 45 isomers with two S-S bridges and 15 isomers with three S-S bridges. During the second stage of folding, the scrambled species reorganize by reshuffling the non-native disulfides to attain the native protein species. Disulfide formation is promoted primarily by oxidized glutathione or cystine, whereas disulfide reshuffling requires a thiol catalyst e.g. reduced glutathione or cysteine or mercaptoethanol.

The effectiveness of thiol reagents in promoting reshuffling is apparently related to their redox potential and each catalyst displays an optimum concentration. Cystine/cysteine is about 10 fold more potent than GSSG/GSH in the process of accumulation of scrambled Hirudins. This difference has been explained by the relative redox potential of the GSSG/GSH (-0.24 V) and Cys-Cys/Cys (-0.22 V) systems. By selecting a combination of optimal conditions (temperature, buffer, salts and redox mixture) the process of folding of Hirudin¹⁻⁴⁹ was accelerated to the extent that it reached completion within 15 min.

In general, the native conformation of a synthetic protein containing several disulfide bonds should form spontaneously under optimum conditions for folding polythiol forms. In many instances, however, even in optimized conditions oxidative foldings mediated by the above redox buffers a considerable amount of byproducts and mismatched forms is produced. This is particularly the case in proteins that tend to form the native conformation only on the surface of specific membranes or under assistance of a specific molecular chaperon (S. Sakakibara Biopolymers, Peptide Science 51, 279, 1999).

Furthermore, despite their widespread use, most of the oxidative foldings of polythiol precursors promoted by air or the GSSG/GSH and Cystine/cysteine redox pairs have been conducted in a manner of trial and error, as clearly demonstrated by folding experiments of synthetic chemokine and chemokine analogues. In fact, while native chemokines and a number of their analogues fold readily, the folded structure being stabilized by two or three disulfide bridges, several analogues do not fold well under the same conditions as the corresponding native molecules resulting in partially folded forms. These observations represent a strong indication that changes in the primary structure of the polythiol precursors may adversely affect the induction of correct local folds (â-turns, polyproline helical motifs etc.) in the polypeptide chains to be folded. Hence, the propensity to fold of many thiol precursors is mainly an intrinsic property of the polypeptide chain rather than a function of the specific oxidation system acting on the molecules.

Enhancement of selected disulfide pairings by adding alcohols, acetonitrile and DMSO to buffers at low ionic strength has been also reported. This strategy involves enhancing the formation of specific disulfide bonds by adjusting electrostatic factors in the medium to favor the juxtaposition of oppositely charged aminoacids that border the selected cysteine residues.

Enzymes such as peptidyl disulfide isomerase (PDI) and prolyl isomerase (PPI) have also been employed as additives to catalyze and modulate disulfide exchange. The time required for folding Hirudin in vitro can be shortened from 10 h to 30 sec if PDI is added to the refolding buffer. In this case, the efficiency of folding in vitro does not differ significantly from that observed in vivo.

Polythiol polypeptide precursors are directly obtained by polypeptide-resin acidolytic cleavage when the cysteine residues are protected by acid-labile groups, e.g. Trt. Alternatively and preferably, polypeptides in which all cysteines are protected by an acid-resistant group, e.g. the acetamidomethyl group (Acm), are first isolated as S-cysteine derivatives by acidolytic peptide-resin cleavage and then the Acm group is eliminated by treatment with Hg(AcO)₂ in acetic acid, followed by the removal of Hg ions by gel filtration in the presence of a large excess of mercaptoethanol.

In both cases, however, several side-reactions have been reported to occur at cysteine and tryptophan residues. The indole ring of tryptophan can be derivatized by mercaptoethanol and cysteine gives a number of side-reactions, the most important being oxidation and alkylation by t-butyl cations during the acidolytic removal of the polypeptide chain from the resin.

Thus, because of the shortcomings of the existing methodologies a need exists for more efficient and more simple processes for folding chemically synthesized polypeptides, and preparing biologically active proteins by chemical synthesis.

The object of the present invention is therefore to provide an efficient, simple and rapid process for folding polypeptides and/or proteins, wherein inter alia the formation of isomers containing mismatched disulfide bridges is minimized, and the use of expensive disulfide-reshuffling reagents such as glutathione or enzymes can be omitted, and which method is repeatable, robust and scalable. These and other objects will be apparent to those of ordinary skill in the art.

A process for the preparation of biologically active proteins is provided, comprising
(a) chemically synthesizing a polypeptide that comprises two or more S-ter-butylthio cysteine residues;
(b) treating said polypeptide with a reducing agent in a folding buffer having a predetermined pH and temperature; and
(c) purifying the obtained folded proteins.
Thus, according to the invention, it has, unexpectedly, been found that S-t-Bu-derivatized cysteines can be deprotected, i.e. loose the S-t-Bu moiety, while forming disulphide bridges with other cysteines when incubated in a proper folding buffer at suitable temperature and pH.

According to the present invention, the reducing agent preferably is free cysteine. An excess of cysteine may be added to the buffer (as for example illustrated in Examples 1-5) or the cysteine may be derived from within the polypeptide (as illustrated in Example 6).

In a preferred embodiment of the process of the invention the folding buffer comprises one or more chaotropic salts, in order to bring the polypeptide in equilibrium conditions which allow natural folding to occur. This can for example be achieved by placing the polypeptide and/or protein in fully denaturating conditions, for example by high concentration of the chaotropic salts, and then diluting the chaotropic salt to a lower concentration for folding. The chaotropic salts are preferably chosen from the group consisting of guanidium chloride and urea, and preferably are present in a concentration of 0.1-1 M during folding.

Preferably, the temperature of the folding buffer lies between 25° and 40°C, more preferably between 27° and 38°C, in order to diminish the changes of peptide degradation, while corresponding to natural body temperatures. Most preferably the temperature is about 37 °C during folding.

According to another preferred embodiment of the process of the invention the folding buffer has a slightly alkaline pH. Preferably, the pH lies between 7 and 9, more preferably between 7 and 8.5 in order to promote the folding process. As may be clear from the above, protein folding is dependent on a complex set of interactions. The reaction with cysteine does for example not occur at acidic pH and higher pH values increase the risk of degradation of the polypeptide.

After folding, the target proteins can be purified by methods well known in the art including anion and cation exchange chromatography, hydrophobic interaction chromatography, reverse phase chromatography, affinity chromatography, Hydrophylic Interaction/Cation Exchange Chromatography (HILIC/CEC), Displacement Chromatography (DC) and Sample Displacement Chromatography (SDM). Most preferably, (reverse phase) high performance chromatography in elution as well as displacement chromaotgrapy are employed.

In a preferred embodiment of the process for producing biologically active proteins, the process comprises the steps of
(a) assembling S-t-butyl-thio cysteine polypeptide on an insoluble polymeric support by stepwise chain elongation;
(b) cleaving said S-t-butyl-thio cysteine polypeptide chain from said support by acidolysis;
(c) purifying the obtained S-t-butyl-thio cysteine polypeptide;
(d) folding the purified S-t-butyl-thio cysteine polypeptide by treating said polypeptides with a molar excess of cysteine in a folding buffer comprising a chaotropic salt, preferably guanidinium chloride, and having an alkaline pH and a temperature of about 37° C; and
(e) purifying the obtained folded proteins by reverse phase High Performance Liquid Chromatography.

In an advantageous embodiment of the process of the invention, the polymeric support is a polyamide or polystyrene-based resin functionalized with the acid labile hydroxymethylphenoxyacetic acid linker, as these supports can be suitably used for fully automatized peptide synthesizers, and generally allow synthesis of long polypeptide chains.

As explained above, the present invention is based on the stepwise solid-phase assembly of S-t-butylthio cysteine polypeptides and the finding that considerable amounts of native, biologically active proteins can be produced by subjecting said polypeptides to fold in the presence of a reducing agent, preferably cysteine, at pH slightly above neutrality and a temperature of about 37°C.

It has surprisingly been found that the production of biologically active proteins is achieved with a high molar excess of cysteine in a procedure which removes the S-t-butyl protective group while allowing disulphide bridge formation. Such procedure is more simple and more efficient than the the procedures described in the prior art for the folding of cysteine-containing polypeptides which are obtained by chemical synthesis. The removal of S-t-but thus occurs within the same step as the folding of the polypeptide.

Alternatively, the same folded material can also be obtained by using a combination of S-t-butyl-thio cysteine and cysteine protected with suitable acid-labile groups at selected positions of the polypeptide chain to force the formation of the appropriate disulfide bonds without extra addition of a reducing agent to the folding buffer. In this case acid-labile groups are removed when the peptide is cleaved from the resin at acidic pH. Free cysteines are thus generated which in turn act as a intramolecular "reducing agent" to allow S-t-butyl removal and disulphide formation (as illustrated in Example 6).

The essence of the present invention is based on:
- the rapid assembly of S-thio-t-butylated polypeptide chains on the polymeric support;
- cysteine-catalyzed thiol-disulfide exchange of the derivatives in slightly alkaline conditions leading to cysteinylated polypeptides that are the macromolecular oxidized forms (protein-S-S-cysteine; polypeptide-S-S-cysteine) of the classical redox Cystine/Cysteine pair;
- the concentration of the oxidized macromolecular form remaining constantly low throughout the folding process so that intermolecular disulfide exchange is minimized; and
- lack of aggregation of misfolded intermediates due to preferential and rapid formation of forms with correct cysteine pairing (native structure).

According to the proces for folding polypeptides of the present invention, for example, in the first step, 10 mg of the S-t-butyl derivative are dissolved at room temperature in 1 ml of a buffer, pH 8.0, comprising 6 M of guanidinium chloride, 10 mM Tris and 0.1 M Na₂HPO₄ and the resulting solution is maintained at room temperature for about 20 min. In the second step, the solution is first diluted 10 fold with water to a pH 7.2, (0.6 M guanidinium chloride, 1.0 mM Tris, 10 mM Na₂HPO₄ and final concentration of the polypeptide derivative 1 mg/ml) and then a strong molar excess of cysteine (about 100-fold over the concentration of the polypeptide or protein derivative) is added under stirring. The temperature is gradually increased to 37°C and maintained constant for about 24 h in order to allow folding of the polypeptide to occur.

The folding process of the present invention results in a highly homogenous product and is applicable, with only minor modifications, to any polypeptide which is produced by solid-phase chemical synthesis as the cysteine thio-t-butyl derivative. In addition, the processes according to the invention have a number of other advantages over the process of the prior art, employing precursor polythiol forms, such as
- the cysteine residues of the chain are not alkylated during acidolytic cleavage of polypeptide-resin ;
- both overoxidation of cysteine to sulphonic acid and oxidation leading to intermolecular disulfide bridge formation do not occur;
- the risk of derivatization of the Trp indole ring by mercaptoethanol, which is necessary to eliminate contaminating Hg ions derived from Acm deblocking with Hg(AcO) ₂, is avoided. In fact the cysteine thiolate in the folding mixture of the present invention does not modifie the Trp at all;
- oxidation-sensitive Met, Trp and Tyr residues are not modified during folding;
- costs of production of the final folded products are generally lower compared to those employing polythiol polpeptides and redox buffers.

It will be apparent to those skilled in the art that, although target proteins are generally prepared in high yield using the process of the present invention, in some cases, e.g. in the case of complex proteins with multiple disulfide bonds, a certain population of intermediate forms that have not completely evolved to the native structure (misfolded species) may remain in solution at equilibrium. Misfolded species can be easily separated from the correctly folded species by RP-HPLC and subjected again to the folding conditions of the present invention to increase the overall yield of the process.

According to the invention the term polypeptide refers to a polymer of amino acids bound together by amide linkages. The term protein refers to the polypeptide species in its three-dimensional form, as occurring in the cells and biological fluids of living organisms. Proteins may for example consist of single folded polypeptide chains or may be complex structures consisting of multiple folded polypeptide chains.

The following Examples and figures are provided to illustrate the present invention and are not intended to limit the invention beyond the limitations set forth in the claims.
Figure 1 shows the HPLC profile before S-t-Butyl removal and folding of hu-I-309 (Example 4).
Figure 2 shows the result of the mass determination of the product of Figure 1.
Figure 3 shows the HPLC profile of deprotected, folded hu-I-309 (Example 4), demonstrating a shorter retention time.
Figure 4 shows the result of the mass determination of the product of Figure 3.
Figure 5 shows the result of the mass determination of the product depicted in Figure 3, after treatment with NEM. No change in mass is observed compared to Figure 4, indicating the absence of free -SH groups.
Figure 6 is a graph of the comparison between the biological activity of recombinant 1-309 and the synthetic, folded 1-309 of the present invention. Biological activity was assessed by binding the chemokine, labelled with ¹²⁵I, to human lymphocytes.
Figure 7 shows the analytical HPLC profile of the protein of Example 5 after folding.
Figure 8 shows the preparative HPLC profile of the folded protein of Example 5.
Figure 9 shows the result of the mass determination of the purified product of Example 5, demonstrating the expected molecular weight.
Figure 10 shows the HPLC profile of the polypeptide of Example 6 before S-t-Butyl removal and folding.
Figure 11 shows the result of the mass determination of the polypeptide of Figure 10 (M=H).
Figure 12 shows the HPLC profile of the protein of Example 6 after folding, demonstrating a shorter retention time.
Figure 13 shows the result of the mass determination of the protein of Figure 12 (M=H), demonstrating the expected molecular weight.

### EXAMPLES

### EXAMPLE 1

### Synthesis and folding of Cys^{10,11,34,50}(S-t-Bu)-hu-TARC (thymus and activation reaulated chemokine).

The 71-amino acid residue chemokine derivative was assembled on a 433 A Peptide Synthesizer (Perkin Elmer/ABI) using Fmoc/t-Bu chemistry and a polystyrene-based resin functionalized with the acid-labile hydroxymethylphenoxyacetic acid linker (Wang resin) on which Fmoc-Ser(t-Bu) was attached by DMAP (4-dimethylaminopyridine)-catalyzed esterification. The degree of substitution was 0.57 mmole/g . The synthesis was conducted on a 0.27 mmole scale using a five-fold excess of Fmoc-amino acids and DCI (N,N'-diisopropylcarbodiimide)/HOBt (1-hydroxybenzotriazole) activating reagents in DMF. The coupling time was about 60 min with spectrophotometric monitoring of Fmoc deprotection.

The four cysteine thiols were protected with S-t-Butyl groups and a maximal protecting scheme was used for all other side-chains : Ser(t-Bu), Thr(t-Bu), Tyr(t-Bu), Asp(O-t-bu), Glu (O-t-Bu), Lys (Boc), Trp (BoC), Asn(Trt), Gln(Trt) and Arg(Pmc). After each coupling, capping with acetic anhydride and DIEA in DMF was carried out.

The resulting polypeptide-resin was treated at room temperature with a freshly prepared mixture of TFA/water/TIS (triisopropylsilane)/phenol (78 :5 :12 :5, v/v/v/w, 10 ml/g resin) for 2.5-3.0 h. The cleaved polypeptide derivative was precipitated by direct filtration of the cleavage mixture into cold methyl-t-butyl ether (MTBE) and the precipitate separated by centrifugation, washed twice with ether and dried in air.

The crude product was then dissolved in diluted acetic acid, lyophilized, redissolved in 50% acetic acid and applied to a Sephadex G-50 column (70x25 cm) using 50% acetic acid as the mobile phase. The collected fractions were analyzed by MALDI-TOF mass spectrometry and those containing the desired polypeptide derivative (MW 8,436.9 Da) were pooled and lyophilized after dilution with water.

The pooled fractions were again dissolved in 50% acetic acid and further purified by loading on a 250x10 mm semipreparative Vydac C₄ column. Samples were eluted at a flow rate of 3 ml/min with a linear gradient of 20-80% B in 60 min, where B was 0.1% TFA in acetonitrile and A 0.1% TFA in water. The detection was done at 280 nm and only the fractions containing the target polypeptide were pooled and lyophilized prior to folding.

Folding of the chemokine derivative purified by RP-HPLC was carried out by first dissolving 10 mg of product in 1mg of 6M GnHCl, 0.1 M Na₂HPO₄ and 10 mM Tris at pH=8.0 and room temperature. After 20 min, the solution was diluted by adding 10 ml of water to the final concentration of 0.6 M GnHCl, 10 mM Na₂HPO₄, 1 mM Tris, pH=7.2 and a peptide concentration of 1mg/ml. Folding was initiated by adding cysteine at a concentration of about 20 mM (about 100 fold molar excess with respect to the peptide concentration) and gradually increasing temperature to 37°C.

The folding reaction, occurring at the constant temperature of 37°C in air, was monitored by RP-HPLC analysis of 25 microliter aliquots of solution acid-quenched with acetic acid on a Waters 2690 Separation Module equipped with a Waters 996 Photodiode Array Detector, using a Vydac C₄ analytical column and a 20-60% acetonitrile gradient in 0.1% TFA/water in 40 min with a flow rate of 1.0 ml/min. 1 microliter of each HPLC peak (corresponding to the folding intermediates of the thiol-disulfide exchange reactions) was collected, mixed with 1 microliter of a saturated solution of sinapinic acid in 1:2 acetonitrile/1% TFA in water, dried under vacuum and analyzed by MALDI-TOF mass spectrometry, using a Voyager-DE spectrometer (Perseptive Biosystem, Framingham, MA) equipped with a nitrogen laser. 78% of folded polypeptide formed after 24 h. The peak whose MW corresponded to that of the folded product was further checked by reaction with N-ethylmaleimide (NEM) to detect the presence of free thiol groups (+125 Da for every SH).

The biological activity of hu-TARC obtained by the methodology of the present invention was performed according to the Imai method (T. Imai et al., J. Biol. Chem., 271, 21514, 1996).

Human T cell lines, Hut78, Hut 102, and Jurkat, as well as fresh monocytes, neutrophils and lymphocytes were assessed for their migration across a polycarbonate filter in response to TARC. No chemotactic response was elicited in monocytes or neutrophyls, neither by TARC prepared by chemical synthesis nor by recombinant TARC. In T cell lines Hut78 and Hut102, synthetic TARC as well as recombinant TARC induced migration with a typical bell-shaped curve with a maximum effect at 100 ng/ml.

### EXAMPLE 2

### Synthesis and folding of Cys^{10,34.50}(S-t-Bu)-hu-TARC and Cys ^{11,34,50} (S-t-Bu)-hu-TARC

The synthesis, purification and folding of cys^{10,34,50} (S-t-Bu)hu-TARC and Cys^{11,34,50} (S-t-Bu)-hu-TARC derivatives has been conducted in the same conditions adopted for Cys^{10,11,34,50} (S-t-Bu) hu-TARC (Example 1), the only difference being the Trt protection at Cys¹⁰ and Cys¹¹, respectively, which was removed concomitantly to cleavage of the polypeptide precursors from the resin. The yields of final folded chemokines was 80% and 79%, respectively.

### EXAMPLE 3

### Synthesis and folding of Cys^{34,50} (S-Bu)-hu-TARC

The synthesis, purification and folding of Cys ^{34,50}(S-Bu) hu-TARC derivative was conducted in the same conditions of the derivatives of Example 1 and 2, except that both Cys¹⁰ and Cys¹¹ were protected by Trt, which was removed during the final resin cleavage by TFA. The yield of folded product was about 75%.

### EXAMPLE 4

### Synthesis and folding of Cys^{10,11,26,34,50,68} (S-t-Bu) -hu-I-309

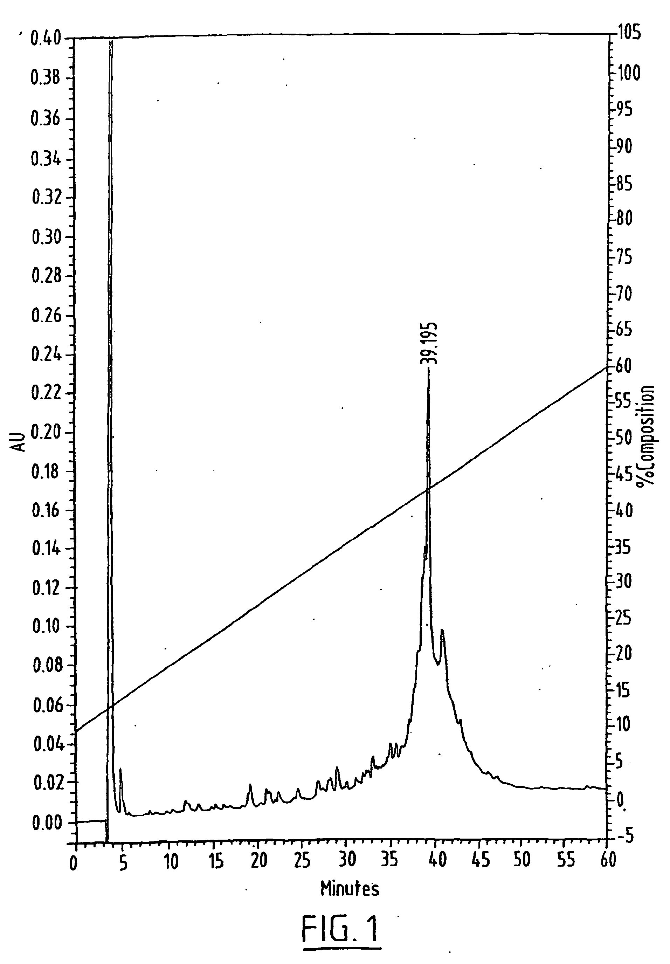

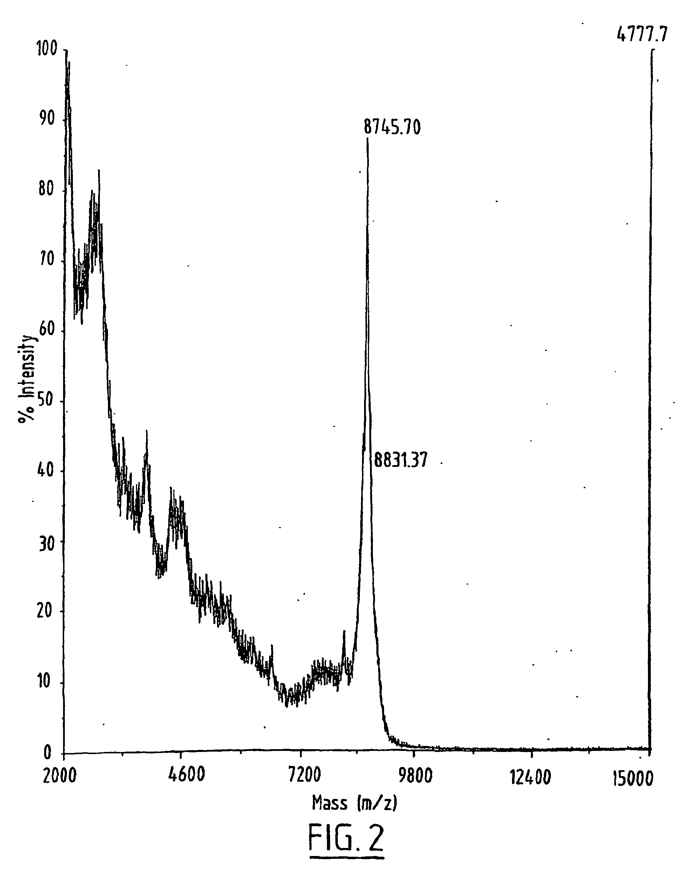

The synthesis of hu-I-309 containing 6 (S-t-Bu) protected cysteines was conducted on a 0.12 mmole scale in the same conditions as in Example 1 using a Fmoc-Lys(Boc) Wang resin (degree of substitution of 0.61 mmol/g). The resulting polypeptide-resin was treated as described for Example 1 and the G50 purified material was further purified by loading on a 250x10 mm Vydac C₁₈ column (as shown in Fig. 1 and 2)

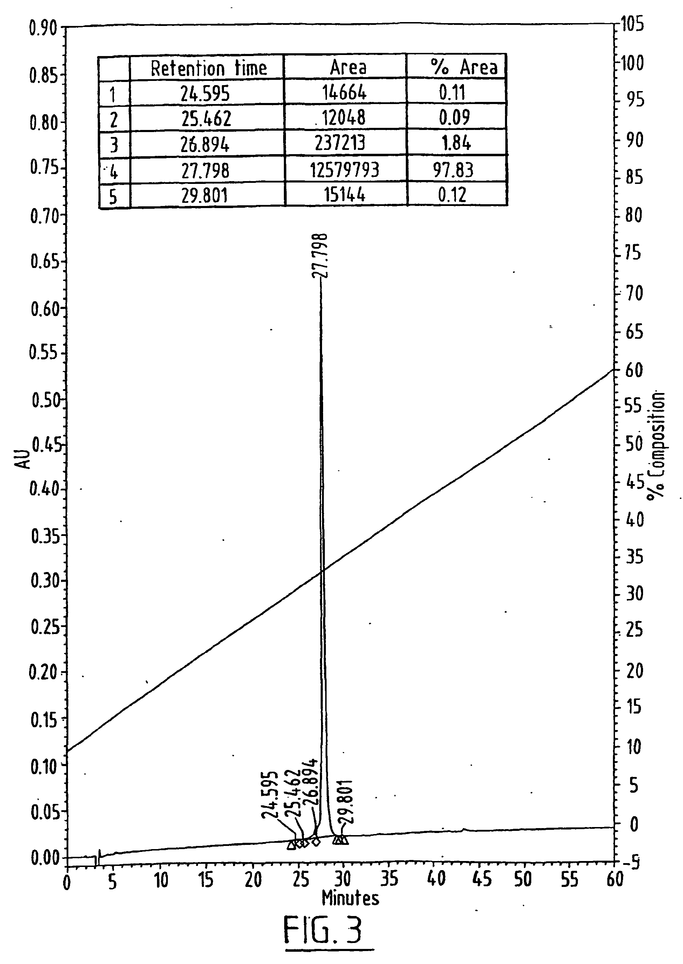

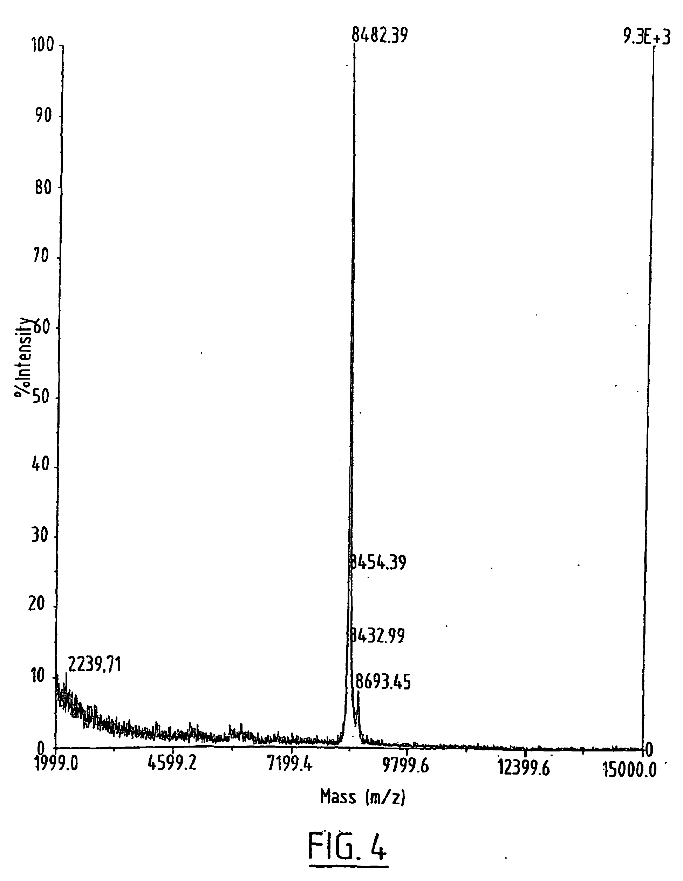

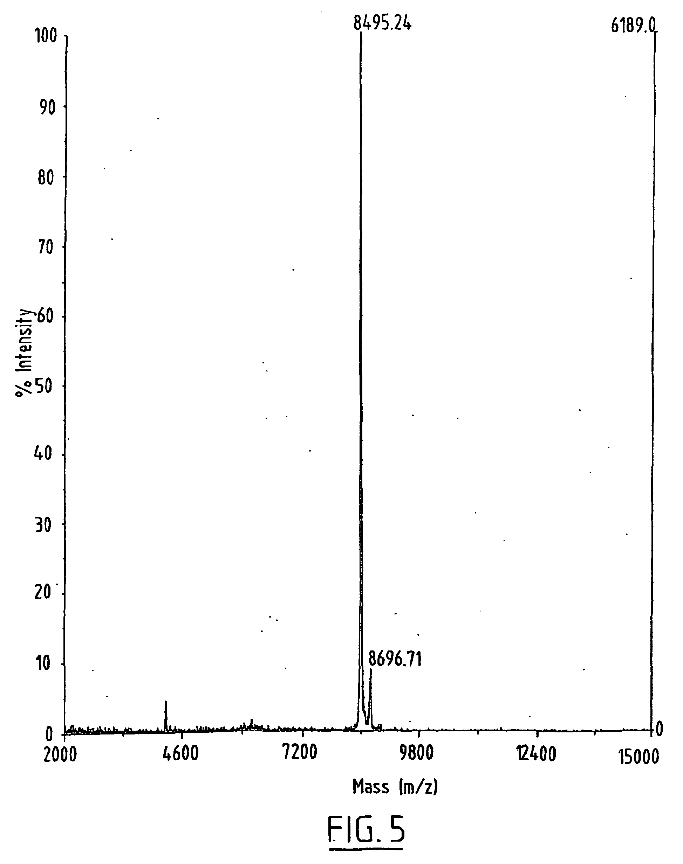

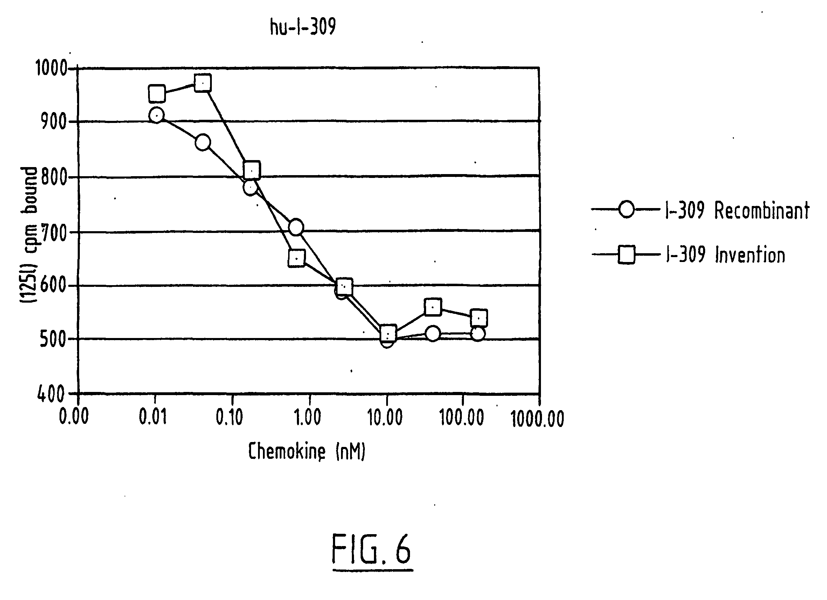

Folding of the chemokine derivative purified by RP-HPLC was carried out by dissolving 65 mg of product in 60 ml of 0.6M GuHCl, 10 mM NaHPO4 and 1 mM Tris at pH 8.0 and adding cysteine at a concentration of 100 fold molar excess with respect to the peptide. The polypeptide solution was left at 37°C for 4 days. After acidulation with TFA the folded material was isolated by RP-HPLC using a 250x10 mm Vydac C₁₈ column (as shown in Fig. 3 and 4). Complete cysteine pairing was checked by mass spectrometry after reaction with N-ethylmaleimide (NEM). No MW increase was observed indicating absence of free thiol groups (as shown in Fig. 5). The yield of final folded chemokine was almost 25%. The synthetic, folded, hu-I-309 showed a biological activity equivalent to recombinant protein (fig. 6).

Analytical chromatography was performed using the following conditions:
Column: C18 250 x 4.6 mm (Vydac#238TP54)
Mobile phase: A = 100% H₂O 0.1%TFA
   B = 100% CH₃CN 0.1%TFA
Gradient: B% composition is reported on the chromatogram
Detector: 214 nm

### EXAMPLE 5

### Synthesis and of Plasmodium vivax C-terminal fragment

The synthesis and purification of Plasmodium vivax circumsporozoite protein (PvCS) 303-372 containing 4. (S-t-Bu) protected cysteines was conducted in the same conditions as in Example 1.

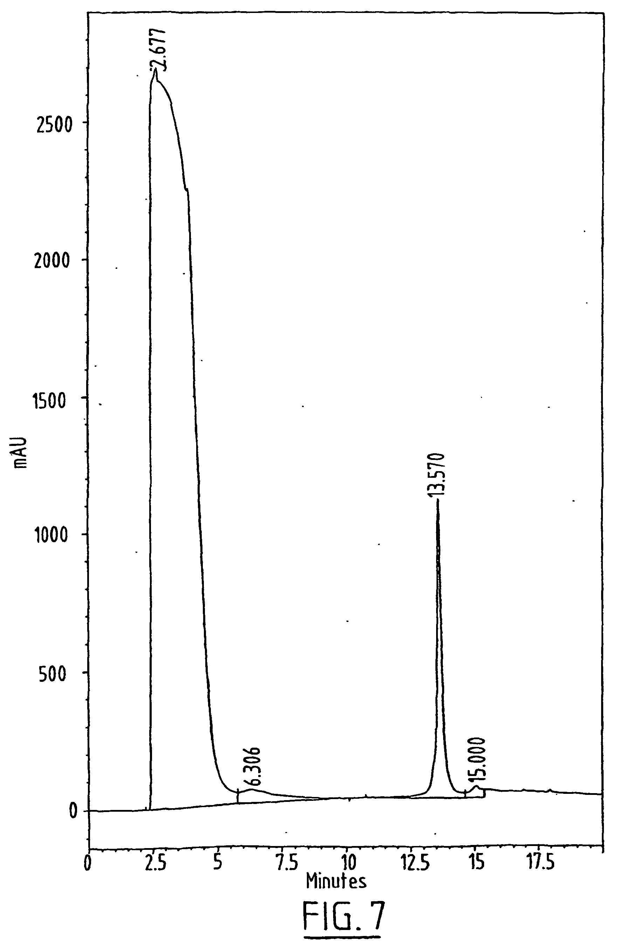

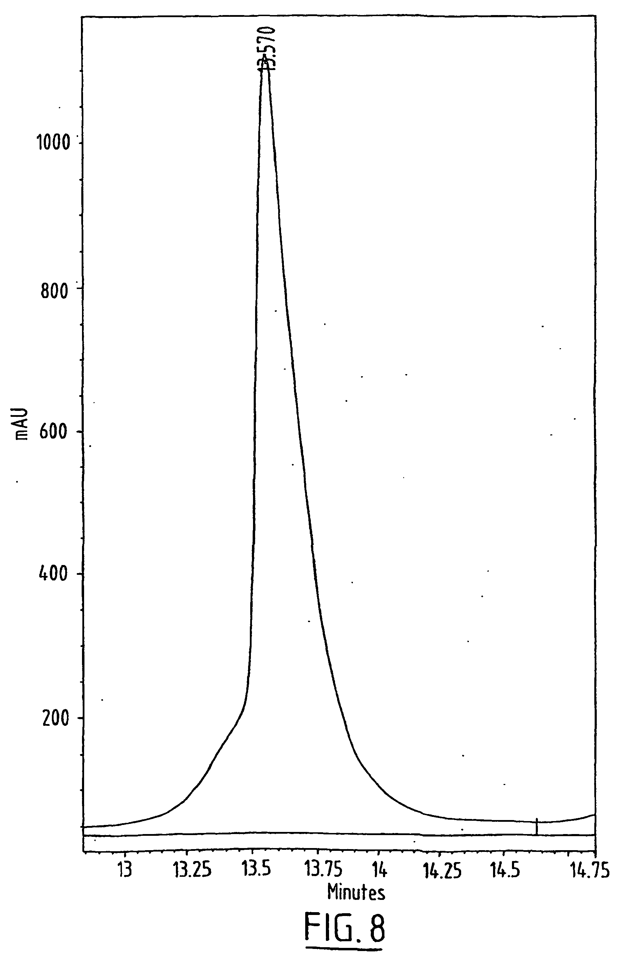

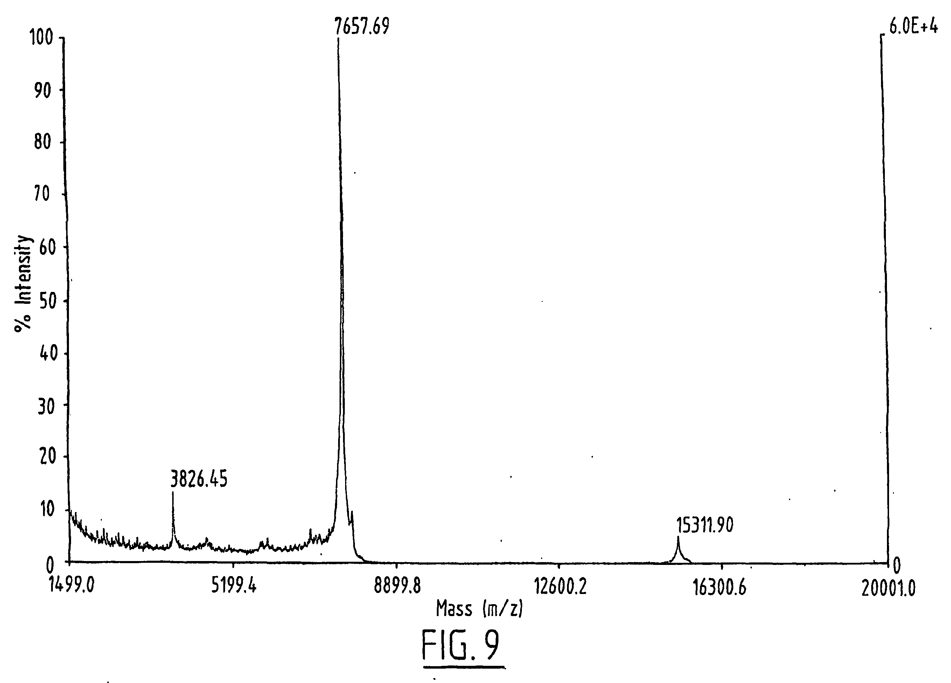

Folding was performed by adding 27 mg of peptide in 2.7 ml of 6 M GuHCl in 0.1 M Tris buffer, pH 8.5. The solution was mixed for 10 min. Then 13.5 ml of 1 mM EDTA, 0.2 M NaCl buffered at pH 8.8 in 0.2 M Tris buffer were added. Finally 10.8 ml of 35 mM cysteine in 1 mM EDTA, 0.2 M NaCl buffered at pH 8.8 in 0.2 M Tris buffer were added. The reaction mixture was brought at 37° C. The folding reaction was followed on reverse phase HPLC to completion (3-6 h) (Fig. 7A) and the reaction stopped by cooling for 5 minutes at 4° C followed by addition of 10% TFA at 4° C to reach a final concentration of 1% TFA (3 ml of 10% TFA). The product was subsequently purified by reverse phase HPLC (Fig. 8) and the mass of the final product determined (Fig. 9). The yield of the final oxidized product was 70-80%.

Analytical chromatography was performed using the following conditions:
Column: C4 250 x 4.6 mm (Vydac#214TP54)
Mobile phase: A = 100% H₂O 0.1%TFA
   B = 100% CH₃CN 0.1%TFA
Gradient: B% composition is reported on the chromatogram
Detector: 214 nm

### EXAMPLE 6

### Large scale synthesis and folding of Plasmodium falciparum C-terminal fragment

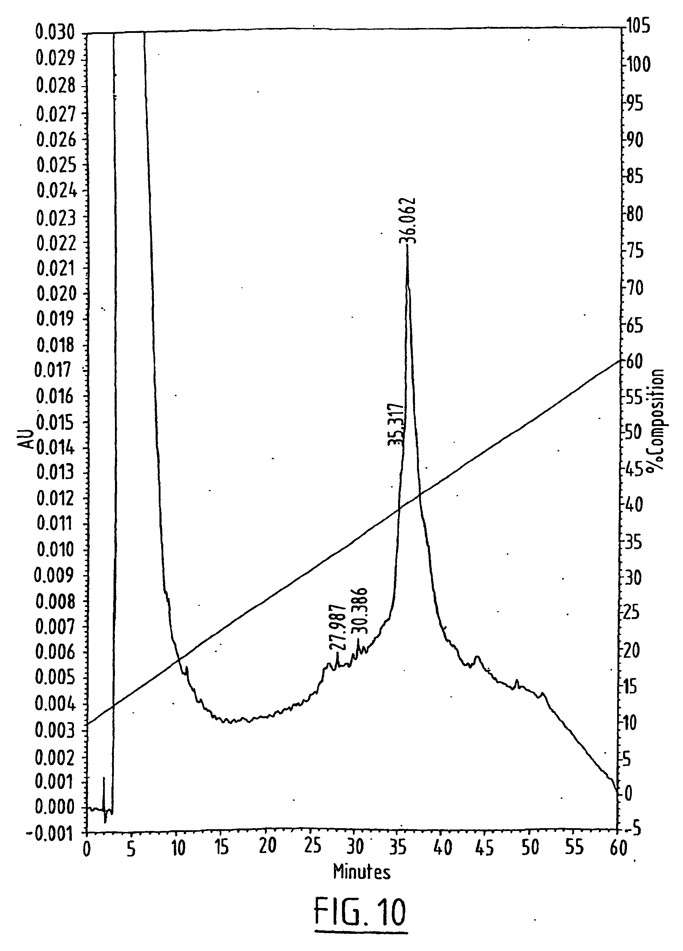

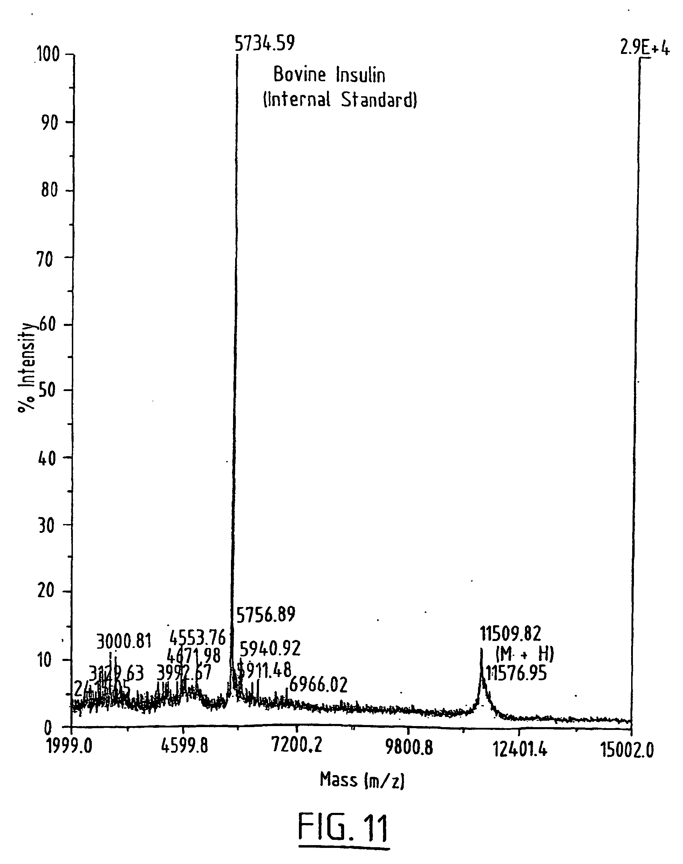

A large scale synthesis and purification of Plasmodium falciparum circumsporozoite protein (PfCS 282-383) containing only 2 (S-t-Bu) protected cysteines out of 4 was conducted in the same conditions as in Example 1 (as shown in Fig. 10 and 11) except for the following.

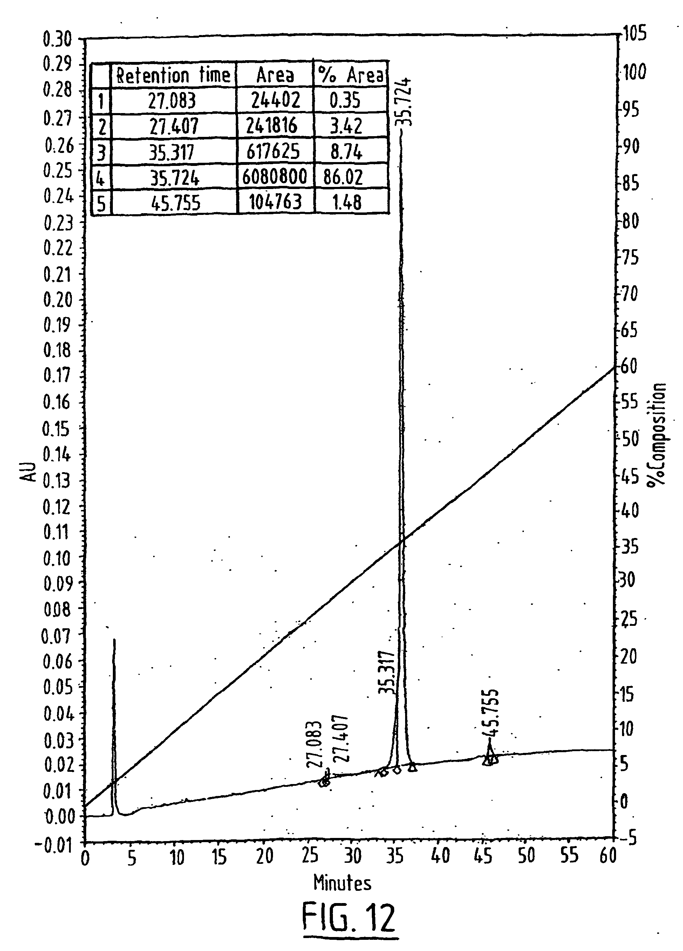

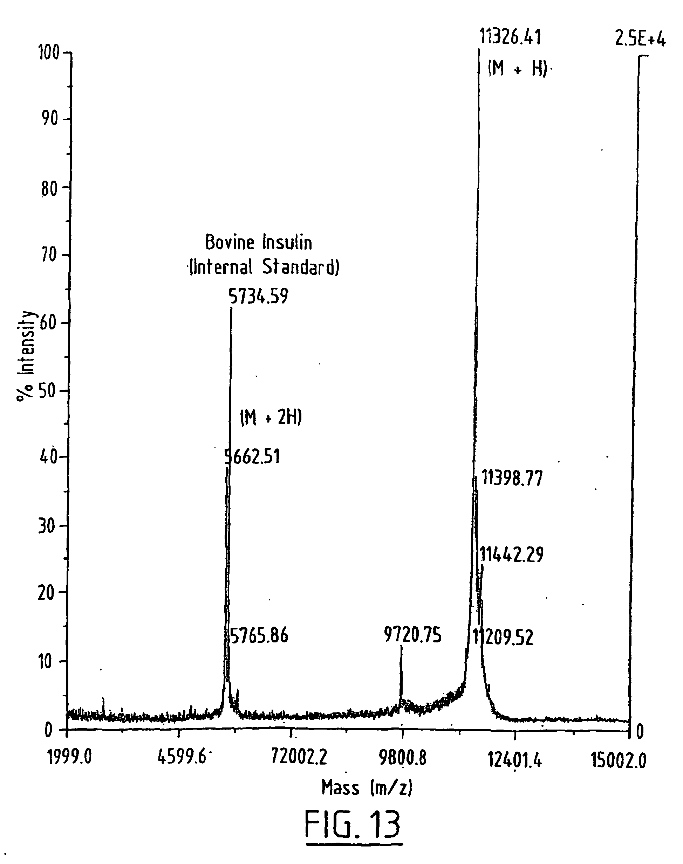

Folding was performed by dissolving 1.1 g of partially purified peptide in 1.0 L of 0.1 M CH₃COONH₄, pH 8.0, without adding free cysteine to the folding buffer. The reaction mixture was maintained at 32° C during 18 h. The product was then purified by reverse phase HPLC (Fig. 12 and 13). The yield of the final oxidized product was almost 37%.

Analytical chromatography was performed using the following conditions:
Column: C18 250 x 4.6 mm (Vydac#238TP54)
Mobile phase: A = 100% H₂O 0.1%TFA
   B = 100% CH₃CN 0.1%TFA
Gradient: B% composition is reported on the chromatogram
Detector: 214 nm

## Claims

1. Process for folding chemically synthesized polypeptides, comprising treating a polypeptide and/or protein that comprises two or more S-ter-butyl-thio-cysteine residues with a reducing agent in a folding buffer having a pH between 7 and 9 at a temperature between 25°C and 40°C.

2. A process for the preparation of biologically active proteins, comprising
(a) chemically synthesizing a polypeptide that comprises two or more S-ter-butyl-thio-cysteine residues;
(b) treating said polypeptide with a reducing agent in a folding buffer having a pH between 7 and 9 at a temperature between 25°C and 40°C; and
(c) purifying the obtained folded polypeptides and/or proteins.

3. The process as claimed in claim 1 or 2, wherein the reducing agent is cysteine.

4. The process as claimed in claim 1 or 2, wherein the reducing agent is an intramolecular reducing agent formed by free cysteines in the polypeptide chains that are generated from cysteines protected with acid-labile groups at selected positions of the polypeptide chain upon cleavage of the polypeptide chain from the resin at acidic pH.

5. The process as claimed in any one of the claim 1-4, wherein the folding buffer comprises one or more chaotropic salts.

6. The process as claimed in claim 5, wherein the chaotropic salts are chosen from the group consisting of guanidium chloride and urea.

7. The process as claimed in any one of the claims 4-6, wherein the chaotropic salts in the folding buffer are present in a concentration of 0.1-1 M.

8. The process as claimed in any one of the claims 1-7, wherein the pH lies between 7 and 8.5.

9. The process as claimed in any one of the claims 1-8, wherein the temperature lies between 27°C and 38°C.

10. The process as claimed in claim 9, wherein the temperature is about 37°C.

11. The process as claimed in claim 2, comprising the steps
(a) assembling S-t-butyl-thio cysteine polypeptide on an insoluble polymeric support by stepwise chain elongation;
(b) cleaving said S-t-butyl-thio cysteine polypeptide chain from said support by acidolysis;
(c) purifying the obtained S-t-butyl-thio cysteine polypeptide;
(d) folding the purified S-t-butyl-thio cysteine polypeptide by treating said polypeptide derivatives with a molar excess of cysteine in a folding buffer comprising a chaotropic salt and having an alkaline pH and a temperature of about 37°C; and
(e) purifying the obtained folded proteins by reverse phase High Performance Liquid Chromatography.

12. The process as claimed in claim 11, wherein the chaotropic salt is guanidinium chloride.

13. The process as claimed in claim 11 or 12, wherein said polymeric support is a polyamide or polystyrene-based resin functionalized with the acid labile hydroxymethylphenoxyacetic acid linker.

## Patentansprüche

1. Verfahren zum Falten chemisch synthetisierter Polypeptide, umfassend: Behandeln eines Polypeptids und/oder Proteins, welches zwei oder mehr S-ter-butyl-thio-Cystein-Reste umfasst, mit einem Reduktionsmittel in einem faltenden Puffer, welcher einen pH zwischen 7 und 9 aufweist, bei einer Temperatur zwischen 25°C und 40°C.

2. Verfahren zum Präparieren biologisch aktiver Proteine, umfassend:
(a) chemisches Synthetisieren eines Polypeptids, welches zwei oder mehr S-ter-butyl-thio-Cystein-Reste umfasst;
(b) Behandeln dieses Polypeptids mit einem Reduktionsmittel in einem faltenden Puffer, welcher einen pH zwischen 7 und 9 aufweist, bei einer Temperatur zwischen 25°C und 40°C; und
(c) Reinigen der erhaltenen gefalteten Polypeptide und/oder Proteine.

3. Verfahren gemäß Anspruch 1 oder 2, wobei das Reduktionsmittel Cystein ist.

4. Verfahren gemäß Anspruch 1 oder 2, wobei das Reduktionsmittel ein intramolekulares Reduktionsmittel ist, welches aus freien Cysteinen in den Polypeptid-Ketten gebildet wird, welche bei Abtrennen der Polypeptid-Kette von dem Harz in saurem pH aus mit Säure-labilen Gruppen geschützten Cysteinen an ausgewählten Positionen der Polypeptid-Kette erzeugt werden.

5. Verfahren gemäß einem der Ansprüche 1 bis 4, wobei der faltende Puffer ein oder mehrere chaotropische Salze umfasst.

6. Verfahren gemäß Anspruch 5, wobei die chaotropischen Salze aus der Gruppe ausgewählt werden, welche aus Guanidin-Chlorid und Harnstoff besteht.

7. Verfahren gemäß einem der Ansprüche 4 bis 6, wobei die chaotropischen Salze in dem faltenden Puffer in einer Konzentration von 0,1 - 1 M vorhanden sind.

8. Verfahren gemäß einem der Ansprüche 1 bis 7, wobei der pH zwischen 7 und 8,5 liegt.

9. Verfahren gemäß einem der Ansprüche 1 bis 8, wobei die Temperatur zwischen 27°C und 38°C liegt.

10. Verfahren gemäß Anspruch 9, wobei die Temperatur ungefähr 37°C beträgt.

11. Verfahren gemäß Anspruch 2, umfassend die Schritte:
(a) Aufbauen von S-t-butyl-thio-Cystein-Polypeptid auf einem unlöslichen Polymer-Träger mittels schrittweiser KettenVerlängerung;
(b) Abtrennen der S-t-butyl-thio-Cystein-Polypeptid-Kette von dem Träger mittels Acidolyse;
(c) Reinigen des erhaltenen S-t-butyl-thio-Cystein-Polypeptids;
(d) Falten des gereinigten S-t-butyl-thio-Cystein-Polypeptids durch Behandeln der Polypeptid-Derivate mit einem molaren Überschuss von Cystein in einem faltenden Puffer, welcher ein chaotropisches Salz umfasst, und einen alkalischen pH und eine Temperatur von ungefähr 37°C aufweist;
(e) Reinigen der erhaltenen gefalteten Proteine mittels Hochleistungs-Flüssigkeits-Chromatographie in inverser Phase.

12. Verfahren gemäß Anspruch 11, wobei das chaotropische Salz Guanidin-Chlorid ist.

13. Verfahren gemäß einem der Ansprüche 11 oder 12, wobei der Polymer-Träger ein Polyamid oder Polystyren-basierter Harz ist, welcher mit dem Säure-labilen Hydroxymethyl-Phenoxyessigsäure-Verbinder/Linker funktionalisiert ist.

## Revendications

1. Procédé pour plier des polypeptides synthétisés chimiquement, comprenant le traitement d'un polypeptide et/ou d'une protéine qui comprend deux ou plusieurs résidus S-ter-butyl-thio-cystéine avec un réducteur dans un tampon de pliage ayant un pH compris entre 7 et 9 à une température comprise entre 25 °C et 40 °C.

2. Procédé pour la préparation de protéines biologiquement actives, comprenant
(a) la synthèse chimique d'un polypeptide qui comprend deux ou plusieurs résidus S-ter-butyl-thio-cystéine
(b) le traitement dudit polypeptide avec un réducteur dans un tampon de pliage ayant un pH compris entre 7 et 9 à une température comprise entre 25 °C et 40 °C ; et
(c) la purification des polypeptides et/ou des protéines pliées obtenues.

3. Procédé selon la revendication 1 ou 2, dans lequel le réducteur est la cystéine.

4. Procédé selon la revendication 1 ou 2, dans lequel le réducteur est un réducteur intramoléculaire formé par des cystéines libres dans les chaînes polypeptidiques qui sont générées à partir des cystéines protégées avec des groupes labiles aux acides en des positions choisies de la chaîne polypeptidique après clivage de la chaîne polypeptidique à partir de la résine à un pH acide.

5. Procédé selon l'une quelconque des revendications 1 à 4, dans lequel le tampon de pliage comprend un ou plusieurs sels chaotropiques.

6. Procédé selon la revendication 5, dans lequel les sels chaotropiques sont choisis parmi le groupe constitué de chlorure de guanidium et d'urée.

7. Procédé selon l'une quelconque des revendications 4 à 6, dans lequel les sels chaotropiques dans le tampon de pliage sont présents en une concentration de 0,1 à 1 M.

8. Procédé selon l'une quelconque des revendications 1 à 7, dans lequel le pH est compris entre 7 et 8,5.

9. Procédé selon l'une quelconque des revendications 1 à 8, dans lequel la température est comprise entre 27 °C et 38 °C.

10. Procédé selon la revendication 9, dans lequel la température est d'environ 37 °C.

11. Procédé selon la revendication 2, comprenant les étapes consistant à :
(a) assembler un polypeptide de S-t-butyl-thio-cystéine sur un support polymérique insoluble par une élongation de chaîne par étape ;
(b) cliver ladite chaîne polypeptidique de S-t-butyl-thio-cystéine à partir dudit support par acidolyse ;
(c) purifier le polypeptide de S-t-butyl-thio-cystéine obtenu ;
(d) plier le polypeptide de S-t-butyl-thio-cystéine purifié en traitant les dérivés dudit polypeptide avec un excès molaire de cystéine dans un tampon de pliage comprenant un sel chaotropique et ayant un pH alcalin et une température d'environ 37 °C ; et
(e) purifier les protéines pliées obtenues par chromatographie liquide haute pression à inversion de phases.

12. Procédé selon la revendication 11, dans lequel le sel chaotropique est le chlorure de guanidinium.

13. Procédé selon la revendication 11 ou 12, dans lequel ledit support polymérique est une résine polyamide ou à base de polystyrène fonctionnalisée avec le lieur d'acide hydroxyméthylphénoxyacétique labile aux acides.
